# EUROPEAN PATENT APPLICATION

(11) **EP 3 424 908 A1**
(43) Date of publication of application: **09.01.2019**
(21) Application number: 17195069.4
(22) Date of filing: 05.10.2017
(51) Int. Cl.: C07D 237/04, A61K 31/50

(54) **PROCESS FOR PREPARATION OF LEVOSIMENDAN**

(30) Priority: 07.07.2017 IN 201721023959
(71) Applicant: Melody Healthcare Pvt. Ltd., Mumbai 400 062, Maharashtra (IN)
(72) Inventor: SINGHANIA,, Harshvardhan, Mumbai 400062 Maharashtra (IN)
(74) Representative: Rees, Kerry

(57) **Abstract**

The present invention discloses a process for preparation of levosimendan. More particularly, the present invention discloses novel process for preparation of (-)-6-(4-aminophenyl)-5-methylpyridazin-3-(2H)-one from racemic 6-(4-aminophenyl)-4,5-dihydro-5-methyl-3-(2H)-pyridazinone using (R)-2-acetamido-3-mercaptopropanoic acid and further converting the (-)-6-(4-aminophenyl)-5-methylpyridazin-3-(2H)-one into levosimendan. The invention further relates to a novel intermediate, (R)-2-acetamido-3-mercaptopropionate salt of (-)-6-(4-aminophenyl)-4,5-dihydro-5-methyl-3-(2H)-pyridazinone of formula (1a) and novel crystalline polymorphic form II of levosimendan.

## Description

### Technical filed:

The present invention relates to a process for preparation of levosimendan. More particularly, the present invention relates to novel process for preparation of (-)-6-(4-aminophenyl)-5-methylpyridazin-3-(2H)-one from racemic 6-(4-aminophenyl)-4,5-dihydro-5-methyl-3-(2H)-pyridazinone and its further conversion into levosimendan. The invention further relates to a novel intermediate, (R)-2-acetamido-3-mercaptopropionate salt of (-)-6-(4-aminophenyl)-4,5-dihydro-5-methyl-3-(2H)-pyridazinone of formula (1a) and novel crystalline polymorph of levosimendan.

### Background and prior art:

Levosimendan is a calcium sensitizer used in the management of acutely decompensated congestive heart failure.

It is marketed under the trade name Simdax, as a 2.5 mg/mL concentrated solution for IV infusion.

The racemic mixture of [[4-(1,4,5,6-tetrahydro-4-methyl-6-oxo-3-pyridazinyl)phenyl]hydrazono]propanedinitrile was first reported in GB 2228004. The levoisomer of [[4-(1,4,5,6-tetrahydro-4-methyl-6-oxo-3-pyridazinyl)phenyl]hydrazono]propanedinitrile, Levosimendan, was first disclosed in US5569657, having the following formula.

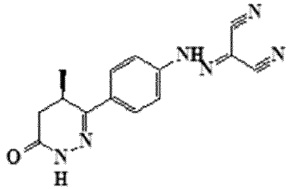

The use of optically-pure (-)-enantiomer of 6-(4-aminophenyl)-5-methylpyridazin-3-(2H)-one, is a key intermediate for the preparation of Levosimendan. The method of preparing (-)-6-(4-aminophenyl)-5-methylpyridazin-3-(2H)-one was disclosed in EP208518, wherein, the separation of pure enantiomers of 6-(4-aminophenyl)-4,5-dihydro-5-methyl-3-(2H)-pyridazinone were carried out using a chiral HPLC column. Further, the isolation of pure levosimendan by passing the racemic mixture over a chiral beta-cyclodextrin stationary phase in reversed phase mode is reported in Chirality, 1996;8(7):511-517, by Wikberg T1, Korkolainen T, Karlsson M (Orion-Farmos, Orion Research, Espoo, Finland). But these processes are not industrially applicable when a large quantity of material is to be isolated.

Subsequently, US6180789 reports a method for preparing optically pure (-)-[[4-(1,4,5,6-tetrahydro-4-methyl-6-oxo-3-pyridazinyl) phenyl] hydrazono] propanedinitrile, which method comprises a) resolving racemic 6-(4-aminophenyl)-4,5-dihydro-5-methyl-3(2H)-pyridazinone by precipitation with a resolving acid in the presence of a solvent b) treating the recovered 6-(4-aminophenyl)-4,5-dihydro-5-methyl-3(2H)-pyridazinone which is enriched in (-) enantiomer with sodium nitrite and malononitrile, c) allowing the resulting [[4-(1,4,5,6-tetrahydro-4-methyl-6-oxo-3-pyridazinyl)phenyl]hydrazono]propanedinitrile which is enriched in (-) enantiomer to mix with acetone, d) removing the precipitate, e) recovering from the mother liquid of step d) the optically substantially pure (-)-[[4-(1,4,5,6-tetrahydro-4-methyl-6-oxo-3-pyridazinyl)phenyl]hydrazono]propanedinitrile by crystallization. The enantiomeric purity of the desired (-)-6-(4-amino phenyl)-5-methylpyridazin-3-(2H)-one product reported in this patent is quite low. Also, the process described in this patent necessitates large volumes of solvent and lower temperature viz., 0-10 °C, which is not feasible for industrial scale up.
US9000158 discloses a process for preparing (-)-6-(4-aminophenyl)-4,5-dihydro-5-methyl-3-(2H)-pyridazinone, which comprises: a) reacting racemic 6-(4-aminophenyl)-4,5-dihydro-5-methyl-3-(2H)-pyridazinone with a chiral tartaric acid derivative to form a first diastereomer salt and a second diastereomer salt, wherein the first diastereomer salt is a salt of (-)-6-(4-aminophenyl)-4,5-dihydro-5-methyl-3-(2H)-pyridazinone and the chiral tartaric acid derivative, and wherein the second diastereomer salt is a salt of (+)-6-(4-aminophenyl)-4,5-dihydro-5-methyl-3-(2H)-pyridazinone and the chiral tartaric acid derivative; and b) isolating (-)-6-(4-aminophenyl)-4,5-dihydro-5-methyl-3-(2H)-pyridazinone from the first diastereomer salt, wherein the chiral tartaric acid derivative is selected from the D- or L-isomer of di-p-anisoyltartaric acid, di-p-tolyl-tartaric acid or O,O'-dibenzoyl-tartaric acid. These resolving agents are very expensive and thus escalate the cost of the process of the levosimendan.

Further, US6180789 reports crystalline polymorphic form I of Levosimendan which is characterized by X-ray diffraction pattern has the following peak positions: 2θ angle(°) 8.7 9.5 12.2 15.4 15.9 17.7 18.4 19.2 20.3 21.4 21.8 23.1 24.6 25.7 27.4.

In view of the problems of the prior art, the inventors felt a need to develop a new process for preparation of Levosimendan by resolution of ± 6-(4-aminophenyl)-4,5-dihydro-5-methyl-3-(2H)-pyridazinone and subsequent conversion of (-)-6-(4-aminophenyl)-4,5-dihydro-5-methyl-3-(2H)-pyridazinone into levosimendan that is simple, economical, eco-friendly and high-yielding.

Another objective of the invention is to provide a novel crystalline polymorph of the levosimendan that can be used in pharmaceutical preparations.

### Summary of the invention

In fulfilling the need of the art, the present inventors provide a cost effective, simple process for preparation of Levosimendan, which process comprises;
a) Resolving ± 6-(4-aminophenyl)-4,5-dihydro-5-methyl-3-(2H)-pyridazinone using (R)-2-acetamido-3-mercaptopropanoic acid in a solvent to obtain (R)-2-acetamido-3-mercaptopropionate salt of (-)-6-(4-aminophenyl)-4,5-dihydro-5-methyl-3-(2H)-pyridazinone of formula (1a);
b) Hydrolysing the (R)-2-acetamido-3-mercaptopropionate salt of (-)-6-(4-aminophenyl)-4,5-dihydro-5-methyl-3-(2H)-pyridazinone of formula (1a) in presence of inorganic base and a solvent to obtain (-)-6-(4-aminophenyl)-4,5-dihydro-5-methyl-3-(2H)-pyridazinone;
c) Reacting the (-)-6-(4-aminophenyl)-4,5-dihydro-5-methyl-3-(2H)-pyridazinone with sodium nitrite and malononitrile under acidic conditions to obtain Levosimendan; and
d) optionally purifying the Levosimendan.

In another aspect, the invention provides novel crystalline polymorphic form which can be used in pharmaceutical preparations.

### Description of drawings:

Figure 1 depicts PXRD of crystalline polymorphic form II of levosimendan in accordance with example 4

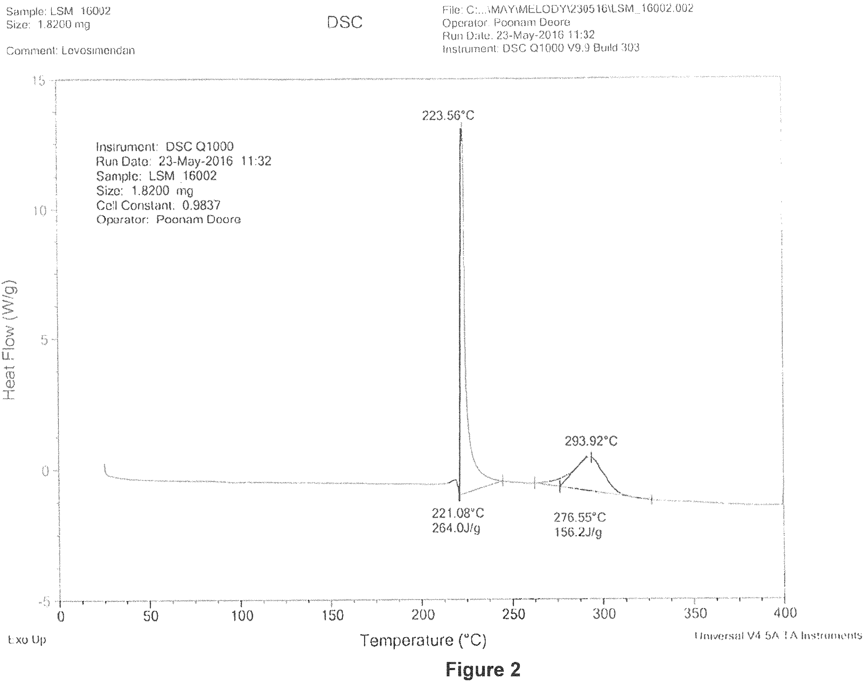

Figure 2 depicts DSC of crystalline polymorphic form II of levosimendan in accordance with example 4.

### Detailed description of the invention:

The invention will now be described in detail in connection with certain preferred and optional embodiments, so that various aspects thereof may be more fully understood and appreciated. Accordingly, in a preferred embodiment, the present invention provides a cost effective, simple process for preparation of Levosimendan, which process comprises;
a) Resolving ± 6-(4-aminophenyl)-4,5-dihydro-5-methyl-3-(2H)-pyridazinone using (R)-2-acetamido-3-mercaptopropanoic acid in an solvent to obtain (R)-2-acetamido-3-mercaptopropionate salt of (-)-6-(4-aminophenyl)-4,5-dihydro-5-methyl-3-(2H)-pyridazinone of formula (1a);
b) Hydrolysing (R)-2-acetamido-3-mercaptopropionate salt of (-)-6-(4-aminophenyl)-4,5-dihydro-5-methyl-3-(2H)-pyridazinone of formula (1a) in presence of inorganic base and a solvent to obtain (-)-6-(4-aminophenyl)-4,5-dihydro-5-methyl-3-(2H)-pyridazinone;
c) Reacting the (-)-6-(4-aminophenyl)-4,5-dihydro-5-methyl-3-(2H)-pyridazinone with sodium nitrite and malononitrile under acidic conditions to obtain Levosimendan; and
d) optionally purifying the Levosimendan.

The resolution of ± 6-(4-aminophenyl)-4,5-dihydro-5-methyl-3-(2H)-pyridazinone is conducted using (R)-2-acetamido-3-mercaptopropanoic acid in mixture of an alcoholic solvent and water preferably at a temperature range of 50 to 70°C under stirring for one to two hrs. After completion of the reaction, the reaction mass is cooled, stirred for another 30 minutes, filtered, washed with water and dried under vacuum to obtain the corresponding diastereomeric salt, (R)-2-acetamido-3-mercaptopropionate salt of (-)-6-(4-aminophenyl)-4,5-dihydro-5-methyl-3-(2H)-pyridazinone(1a).

The alcoholic solvent may be selected from methanol, ethanol and isopropanol. In a preferred embodiment, the alcoholic solvent is ethanol.

The process of resolution step is shown in scheme 1 below.

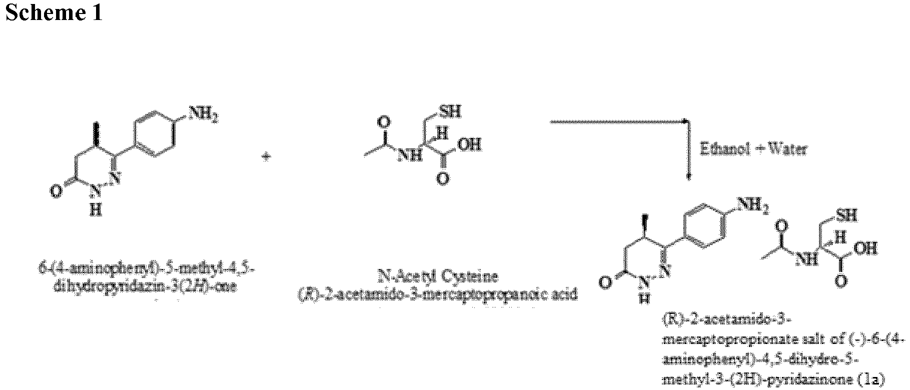

In the next stage, the (R)-2-acetamido-3-mercaptopropionate salt of (-)-6-(4-aminophenyl)-4,5-dihydro-5-methyl-3-(2H)-pyridazinone(1a) thus obtained is taken in water and adjusted the pH of the solution between 8 to 9 by the addition of an inorganic base such as potassium carbonate or ammonia solution under stirring to liberate the (-)-6-(4-aminophenyl)-4,5-dihydro-5-methyl-3-(2H)-pyridazinone free base. The free base is further extracted into a halogenated hydrocarbon solvent, distilled the solvent followed by addition of 1, 4, dioxane, heated, cooled to RT, filtered. The filtrated is collected and distilled out the dioxiane, added ethyl acetate filtered and dried under vacuum at 50° C. to obtain solid (-)-6-(4-aminophenyl)-4,5-dihydro-5-methyl-3-(2H)-pyridazinone. The reaction can be conducted at ambient temperature. The ambient temperature is typically a temperature range of 25-30° C.

The halogenated hydrocarbon solvent is selected from methylene dichloride, ethylene dichloride etc. In a preferred embodiment, the solvent is methylene dichloride.

The process is shown in scheme 2 below.

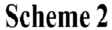

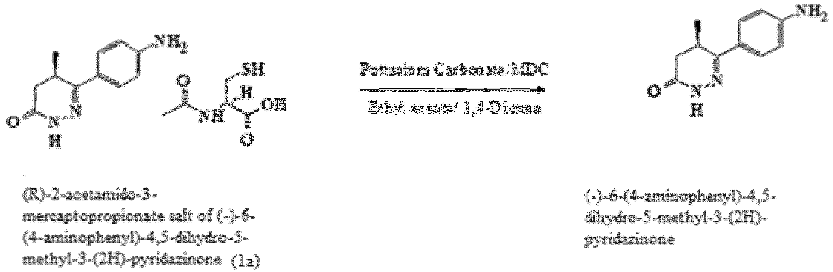

In the final stage, the (-)-6-(4-aminophenyl)-4,5-dihydro-5-methyl-3-(2H)-pyridazinone free base is subjected to diatozation by treatment with sodium nitrite in mineral acid and then reacted with malononitrile under stirring at room temperature. The pH of the suspension is adjusted to 6.0 with sodium acetate solution and filtered the solution to obtain levosimendan with good yield and purity. The process is shown in scheme 3.

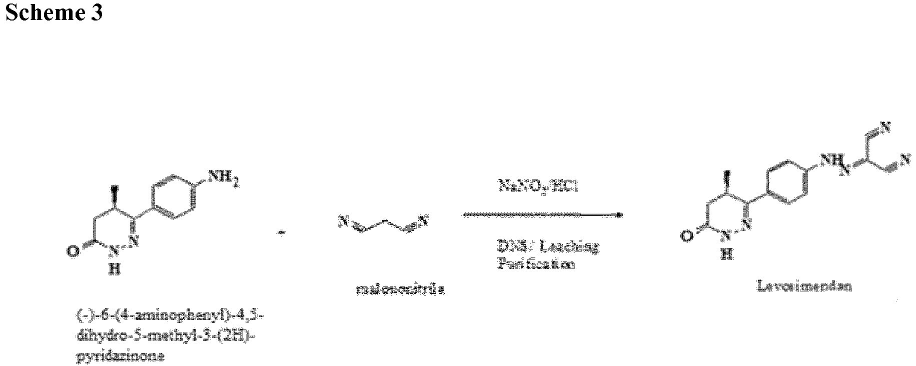

In a further embodiment, the levosimendan thus obtained is purified from mixture of ethanol and diethyl ether in 3:1 ratio to obtain crystalline polymorphic form of Levosimendan designated herein after polymorphic form II.

Accordingly, the invention provides a process for preparation of levosimnendan polymorphic form II which process comprises;
a) dissolving levosimendan in mixture of ethanol and diethyl ether in about 3:1 ratio to obtain a solution;
b) heating the solution of step a) at a temperature of 50-60 °C under stirring followed by cooling the solution to 20-30°C temperature; and
c) isolating the crystalline polymorphic form II of levosimendan by filtration of the solution.

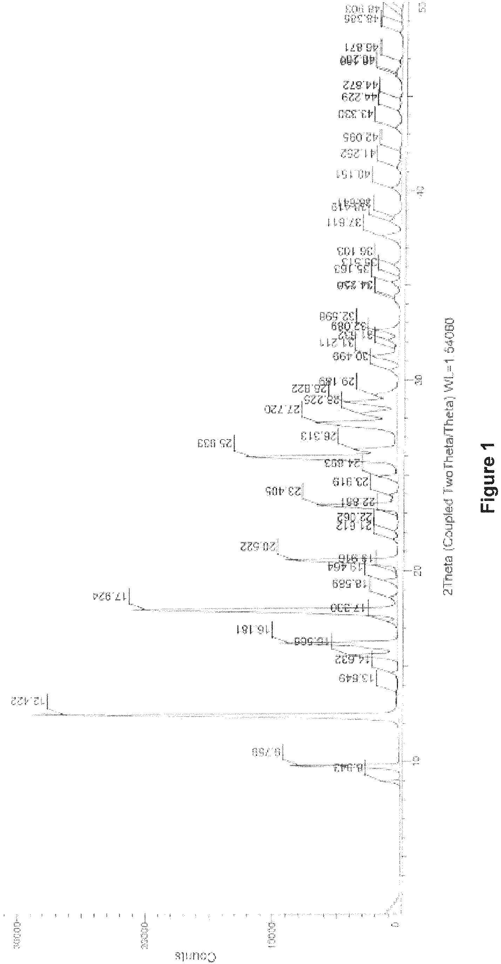

The PXRD pattern shows at least one characteristic peak at about 12.42 degrees2 theta. More particularly the PXRD pattern has a total of 50 peaks having characteristic peaks at 9.75; 12.42; 15.56; 16.18; 17.92; 20.52; 23.40; 25.93; 27.72; 28.22, 28.82 ± 0.2 degrees two theta, as shown in Figure 1.

The crystalline 'Form II' of Levosimendan has X-ray powder diffraction pattern is substantially as shown in the Figure 1 and the characteristic peaks with their 2 theta value and corresponding d spacing are listed in the table given below.

**Table 1**

| 2-theta values in degrees | d spacing values |
|---|---|
| 9.75, | 9.055 |
| 12.42, | 7.119 |
| 15.56, | 5.688 |
| 16.18 | 5.473 |
| 17.92 | 4.944 |
| 20.52 | 4.324 |
| 23.40 | 3.797 |
| 25.93 | 3.433 |
| 27.72 | 3.215 |
| 28.22 | 3.159 |
| 28.82 | 3.095 |

The polymorphic form II of levosimendan is further characterized using Differential scanning calorimetry (DSC) thermogram. The DSC of the polymorphic form II exhibits characteristic endotherm at about 221°C. DSC was measured in a TA Q100 instrument using a standard open pan. The weight of samples was about 2 mg and the samples were scanned at a heating rate of 10°C/min from 0 to 400°C under a nitrogen atmosphere.

The novel crystalline polymorphic form II of levosimendan according to the present invention is more stable during its shelf-life and hence provides desired and consistent therapeutic effect when prepared as a medicament.

The novel crystalline polymorphic form II of levosimendan according to the present invention is more soluble and thus advantageously suitable for pharmaceutical preparations over the polymorphic form 1.

Accordingly, in yet another embodiment, the invention provides pharmaceutical compositions comprising crystalline polymorphic form II of levosimendan having characteristic peaks at 9.75; 12.42; 15.56; 16.18; 17.92; 20.52; 23.40; 25.93; 27.72; 28.22, 28.82 ± 0.2 degrees two theta, in association with one or more commonly used pharmaceutical excipients/carriers.

In a further embodiment, the invention provides pharmaceutical compositions comprising crystalline polymorphic form II of levosimendan having characteristic peaks at 9.75; 12.42; 15.56; 16.18; 17.92; 20.52; 23.40; 25.93; 27.72; 28.22, 28.82 ± 0.2 degrees two theta, optionally in combination with polymorphic form I, along with one or more commonly used pharmaceutical excipients/carriers.

The pharmaceutical compositions can be prepared in the form of oral or injectable forms. Selection of particular excipients and the amounts to use in the preparation of the particular dosage form can be readily determined by the formulation scientist based upon experience considering the standard procedures and reference works in the related area. The pharmaceutical compositions can be formulated into dosage forms according to conventional methods known in the art.

In yet another embodiment, the invention provides novel disatereomeric salt of 6-(4-aminophenyl)-4,5-dihydro-5-methyl-3-(2H)-pyridazinone viz., (R)-2-acetamido-3-mercaptopropionate salt of (-)-6-(4-aminophenyl)-4,5-dihydro-5-methyl-3-(2H)-pyridazinone of formula (1a).

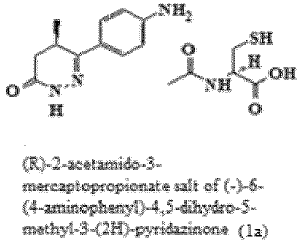

The following examples, which include preferred embodiments, will serve to illustrate the practice of this invention, it being understood that the particulars shown are by way of example and for purpose of illustrative discussion of preferred embodiments of the invention.

### Examples

### Example 1

### Resolution of racemic 6-(4-aminophenyl)-4,5-dihydro-5-methyl-3-(2H)-pyridazinone

Racemic 6-(4-aminophenyl)-4,5-dihydro-5-methyl-3-(2H)-pyridazinone (100.00 gms,0.49 moles) and ethanol (1500.00 ml,15.00 vol) were added to a flask and stirred at 65° C for 30 minutes. Further (R)-2-acetamido-3-mercaptopropanoic acid (226.00gms, 1.38 moles) and water (1500.00 ml, 15.00 vol) were added and stirring was continued for 1 hour. The reaction mass was cooled and stirred for another 30 minutes. Then the mass was filtered, washed with water and dried under vacuum to obtain the corresponding diastereomeric salt, (R)-2-acetamido-3-mercaptopropionate salt of (-)-6-(4-aminophenyl)-4,5-dihydro-5-methyl-3-(2H)-pyridazinone of formula (1a).
**Yield: 90-95%**
**Purity: 85-90 %(Chiral purity)**

### Example 2

### Preparation of (-)-6-(4-aminophenyl)-4,5-dihydro-5-methyl-3-(2H)-pyridazinone

The (R)-2-acetamido-3-mercaptopropionate salt of (-)-6-(4-aminophenyl)-4,5-dihydro-5-methyl-3-(2H)-pyridazinone (180.00 gm, 0.49 moles), and water (1000.00 ml, 5.55 vol) were added to a reaction vessel and stirred at 25-30° C. for 30 minutes. The pH of the resulting solution was adjusted to 8 to 9 by adding potassium carbonate solution (80.00 gm, 0.57 moles of potassium carbonate in 800.00 ml, 10.00 vol. of water) and stirring was continued for 30 minutes. After completion of reaction, the product was extracted into MDC (1000.00 ml, 5.55 vol), MDC was distilled out, added Dioxane(120.00 ml,0.66 vol), heated and further cooled to RT, filtered and collected the filtrate, disttilled out the dioxane, added ethyl acetate(50.00 ml,0.27 vol.), filtered and dried under vacuum at 50°C to obtain solid (-)-6-(4-aminophenyl)-4,5-dihydro-5-methyl-3-(2H)-pyridazinone.
**Yield: 20.00-25.00 %**
**HPLC purity: NLT 99.00 %**
**Chiral purity: NLT 98.00%**

### Example 3

### Preparation of Levosimendan

To a solution of (-)-6-(4-aminophenyl)-4,5-dihydro-5-methyl-3-(2H)-pyridazinone (15.00 gms,0.07 moles in 590.00 ml of water) was added dilute hydrochloric acid, (39.00 ml ,0.32 moles (on 100% basis) dilute sodium nitrite (6.00 gms,0.08 moles in 39.00ml water i.e. 2.60 vol.) and stirred. After 10 minutes, malononitrile solution (4.70 Gms, 0.07 moles in 39.00 ml water i.e. 2.60 vol.) was added. The solution was stirred for 1 hour at room temperature. The pH of the suspension was adjusted to 6.0 with sodium acetate (180.00 Gms, 30.00 moles in 800.00 ml water i.e.4.44 vol) solution. The suspension was filtered, washed with copious amounts of water followed by ethanol and then dried to obtain solid levosimendan. The Levosimendan is purified from ethanol (90.00ml, 6.00 vol.) and diethyl ether (30.00 ml, 2.00 vol.).
**Yield: 60.00-70.00%**
**HPLC Purity: NLT: 99.00%**
**Chiral purity: NLT: 99.00%**

### Example 4

### Preparation of Levosimendan polymorphic form II

The levosimendan (gms) from example 3 was taken in ethanol (90ml) and diethyl ether (30ml), heated at a temperature of 50-60 °C under stirring, followed by cooling the solution to 20-30°C to obtain Levosimendan polymorphic form II, which isolated.

The PXRD pattern of the Form II thus obtained is shown in figure 1 and the DSC is shown is figure II.
**Yield: 60.00-70.00%**
**HPLC Purity: NLT 99**.**00**%
**Chiral purity: NLT 99**.**00**%

## Claims

1. A cost effective process for preparation of Levosimendan, which process comprises;
a) Resolving ± 6-(4-aminophenyl)-4,5-dihydro-5-methyl-3-(2H)-pyridazinone using (R)-2-acetamido-3-mercaptopropanoic acid in a solvent to obtain (R)-2-acetamido-3-mercaptopropionate salt of (-)-6-(4-aminophenyl)-4,5-dihydro-5-methyl-3-(2H)-pyridazinone of formula (1a);
b) Hydrolysing the (R)-2-acetamido-3-mercaptopropionate salt of (-)-6-(4-aminophenyl)-4,5-dihydro-5-methyl-3-(2H)-pyridazinone of formula (1a) in presence of an inorganic base in a solvent to isolate (-)-6-(4-aminophenyl)-4,5-dihydro-5-methyl-3-(2H)-pyridazinone; and
c) Reacting the (-)-6-(4-aminophenyl)-4,5-dihydro-5-methyl-3-(2H)-pyridazinone with sodium nitrite and malononitrile under acidic conditions to obtain Levosimendan and
d) optionally purifying the Levosimendan.

2. The process according to claim 1, wherein, the reaction of step a) is conducted at a temperature of 50 to 70°C.

3. The process according to claim 1, wherein, the solvent in step a) is a mixture of ethanolic solvent and water.

4. The process according to claim 1, wherein, the reaction of step b) is conducted at ambient temperature.

5. The process according to claim 1, wherein, the solvent of step b) is methylene dichloride.

6. The process according to claim 1, wherein, the inorganic base in step b) is selected from ammonia or potassium carbonate.

7. The process according to claim 1, wherein, the purification of levosimendan is carried out from mixture of ethanol and diethyl ether in 3:1 ratio.

8. A novel crystalline polymorphic form II of Levosimendan which is **characterized by** a powder X-Ray diffraction pattern (PXRD) having peaks at 9.75; 12.42; 15.56; 16.18; 17.92; 20.52; 23.40; 25.93; 27.72; 28.22, 28.82 ± 0.2 degrees two theta.

9. Pharmaceutical compositions comprising Levosimendan polymorphic form II which is **characterized by** a powder X-Ray diffraction pattern (PXRD) having peaks at 9.75; 12.42; 15.56; 16.18; 17.92; 20.52; 23.40; 25.93; 27.72; 28.22, 28.82 ± 0.2 degrees two theta optionally in association with one or more pharmaceutical carriers/excipients.

10. A process for preparation of levosimnendan polymorphic form II according to claim 8, which process comprises;
a) dissolving levosimendan in mixture of ethanol and diethyl ether in 3: 1 ratio to obtain a solution;
b) heating the solution of step a) at a temperature of 50-60°C under stirring followed by cooling the solution to 20-30°C temperature; and
c) isolating the crystalline polymorphic form II of levosimendan by filtration of the solution.

11. (R)-2-acetamido-3-mercaptopropionate salt of (-)-6-(4-aminophenyl)-4,5-dihydro-5-methyl-3-(2H)-pyridazinone of formula (1a).
